# EUROPEAN PATENT APPLICATION

(11) **EP 1 609 487 A1**
(43) Date of publication of application: **28.12.2005**
(21) Application number: 05105057.3
(22) Date of filing: 09.06.2005
(51) Int. Cl.: A61L 2/20, B65B 55/04, B65B 55/10

(54) **Aseptic composition for sterilisation using ozone in liquid carbon dioxide**

(30) Priority: 15.06.2004 US 579700 P; 31.08.2004 US 930628
(71) Applicant: L'AIR LIQUIDE, SOCIETE ANONYME POUR L'ETUDE ET L'EXPLOITATION DES PROCEDES GEORGES CLAUDE, 75007 Paris (FR)
(72) Inventor: Fisher, Steven A., Brookfield, IL 60513 (US); Stiener, Edward F., Downers Grove, IL 60516 (US); Sundaram, Meenakshi, Burr Ridge, IL 60527 (US); Yuan, James, Naperville, IL 60565 (US)
(74) Representative: Mellul-Bendelac, Sylvie Lisette

(57) **Abstract**

The use of ozone in liquid carbon dioxide as a sterilant for use in aseptic packaging. The low temperature provided by liquid CO2 further eliminates microbiological hazards. The combination of these two chemicals will enable the aseptic industry achieve higher process efficiencies and improved sterilization without the drawbacks currently seen with the use of hydrogen peroxide.

## Description

The term aseptic packaging in generic terms means performing microbiological sterilization of product and package separately and then filling the package with the product under sterile condition, such as an aseptic atmosphere. The asceptic packaging, and its extended term ultra clean, extend to a wide array of packaging material, shapes and forms. This includes cups, bricks, pouches, bottles and jars. These packages are generally made of paper, plastic, etc. Many foodstuffs, such as drinks, milk, milk products, sauces, soups, etc., are aseptically packaged to increase their shelf-life and product quality.

The technology for commercial aseptic packaging has been available for over 50 years, and has been used with great acceptance in Europe since the early 1960's. This process was not approved for use in the United States until 1981. Initially only used for single serving milk and juice containers, recently this process has grown in popularity. This is due to the reduction of mass produced aseptic packaging equipment, and the increase in energy costs in the past 5 to10 years. It uses much less energy to run an aseptic plant, and the storage costs are greatly reduced since there is no need to refrigerate. It is expected that the 535 aseptic packaging installations in the United States alone produce between 15 and 20 billion packages a year. Today, aseptic packages range in size from small consumer 'juice boxes' to multi-thousand gallon commercial containers of orange juice or tomato paste.

With aseptic packaging, as opposed to traditional methods such as canning, the package is sterilized, and the sterilized food is introduced into this sanitized package in a sterile environment, then sealed hermetically. This seal is proof against light and oxygen. No artificial preservatives or subsequent refrigeration is needed. These packages are shelf stable for up to a year or longer.

In the traditional canning process, the food is introduced into a non-sterilized container, then heated. The container is maintained at a high temperature for a sustained length of time (typically 20 to 50 minutes), effectively cooking the food within the container as it disinfects the container itself.

Thus, the advantages of aseptic packaging over traditional packaging systems are:
- Food safety
- Shelf stability independent of ingredients
- Ability to process thermally sensitive products
- More robust process
- Thermally process a product independent of package size
- More precise control over process
- Flexible use of containers varying in design/materials
- Produce a product of improved sensory quality (color, taste, texture, etc.)
- Product does not require refrigeration, resulting in energy savings in storage
- Improved nutrient retention

The most commonly used process involves sterilizing with concentrated hydrogen peroxide. Hydrogen peroxide provides several benefit such as bactericidal effect, environmental compatibility and ease with which the technical process can be implemented. Hydrogen peroxide offers the advantage that the only by-products are water and oxygen. The FDA approved hydrogen peroxide to be used as a sterilant for package sterilization in 1981. Presently in almost all commercial equipment, the packaging is sterilized by hydrogen peroxide.

A variety of different machines are currently using this process. These include SIG Combibloc machines for the filling of prefolded "bricks", Stork machines for the filling of plastic bottles, Hamba machines for the filling of small plastic packaging, Tetra Pak machines for filling the "brick" packs, and Bosh machines for filling of small plastic packages.

Two different techniques are broadly used to apply hydrogen peroxide on the surface of packaging depending upon the type commercial installation and product used. These are either the soaking of the package material in a bath of hydrogen peroxide, or the spraying of the package material with hydrogen peroxide.

The method of soaking in a bath of hydrogen peroxide is generally applied to Tetra Pak machines and the like. The hydrogen peroxide bath is commonly heated between about 45 °C to about 80°C. The packaging material then comes in contact with peroxide solution in the bath. Also additional details are in European patent application (EP 0950608 A1), which is incorporated by reference. In this process decomposition can occur when impurities from the packaging material accumulate in the bath and catalyze the hydrogen peroxide. Another problem occurs as the water evaporates and the hydrogen peroxide concentration of the bath increases.

Stabilization of the hydrogen peroxide is an important factor in this type of system. If the hydrogen peroxide stability is too high, the hydrogen peroxide concentration of the bath will increase. If the hydrogen peroxide is too low, the hydrogen peroxide concentration of the bath will decrease. In the immersion bath method, the bath concentration has to be boosted or the fluid changed regularly to maintain adequate germicidal effect. There is higher-level risk of certain portion of material not treated as well as frequent changing of peroxide solution

The method of spraying hydrogen peroxide is a more common method. This method is used in Combibloc machines and the like. In this method, hydrogen peroxide is sprayed or vaporized at higher temperature, typically about 120°C to about 250°C. A problem with this system is that deposits can remain in the heating system and may plug the spray nozzles. Therefore, it is necessary to use a grade of hydrogen peroxide with a dry residue as low as possible. However, even the commercially available hydrogen peroxide with the lowest dry residue will still deposit on the order of 15 to 20 mg of residue for every kg of hydrogen peroxide.

The use of hydrogen peroxide in this application results in a number limitations.
- First, slower machine speeds. In order to achieve the desired contact time between the hydrogen peroxide bath and the packaging material, the rate at which the material can flow through the bath is limited.
- Second, the potency of hydrogen peroxide to kill microorganisms is limited in comparison to other more powerful oxidants such as chlorine, chlorine dioxide, ozone, etc.
- Third, in the spray method, the peroxide is sprayed or nebulized stepwise via a nozzle to disinfect the receptacle. The spray nozzles tend to plug up frequently, thereby increasing concerns that certain portion of material may not be adequately treated with the hydrogen peroxide.
- Fourth, there is a potential problem with residue. Several stages of heating typically follow the treatment step, to evaporate excess hydrogen peroxide by using sterile hot air. Temperature of 250 °C to 350 °C are usual in the hydrogen peroxide evaporation unit. As a result of a high throughput, deposits occur which are hard to be removed.
- Fifth, the increasing water availability for microbial growth. Hydrogen peroxide is a mixture of water and peroxide based on concentration. The presence of water increases the possibility for microbiological growth if the potency of peroxide is reduced in the bath due to continuous operation.
- And finally sixth, adequate germicidal effect. The hydrogen peroxide strength in the immersion bath is gradually reduced due to; the disinfecting process itself, impurities that are introduced into the bath by the packaging material, and higher temperatures.

For the foregoing reasons, a need exists within the industry for a system that will allow for higher machine and packaging speeds, while maintaining or improving the sterilization of the packaging material. A need also exists within the industry for a system that will reduce or eliminate the current problem with spray nozzle plugging and residues resulting from the dried sterilant. A need exists within the industry for a system that will improve the germicidal value of the sterilization fluid.

The present invention is directed to a method that satisfies the need in society in general for a system that will allow for higher machine and packaging speeds, while maintaining or improving the sterilization of the packaging material. The present invention is also directed to a system that satisfies the need within the industry for a system that will reduce or eliminate the current problem with spray nozzle plugging and residues. The present invention is also directed to a system that satisfied the need within the industry for a system that will improve the germicidal value of the sterilization fluid.

This method utilizes ozone in a liquid, other than water, such as carbon dioxide as a sterilant for use in aseptic packaging. Ozone is proven to be more effective in killing microorganisms than hydrogen peroxide, due to its higher oxidation potential. The low temperature provided by liquid CO2 further eliminates microbiological hazards. The combination of these two chemicals will help the aseptic industry achieve higher process efficiencies and improved sterilization without the drawbacks of the use of hydrogen peroxide

In one aspect of the present invention, an antimicrobial composition comprising a liquid, other than water, and an effective amount of a sterilant is provided. In another aspect of the present invention, the liquid is liquid carbon dioxide. In another aspect of the present invention, the liquid is a cryogenic fluid. In another aspect of the present invention the cryogenic fluid is selected from the group consisting of liquid oxygen, liquid nitrogen, liquid argon, liquid air, or any combination. In another aspect of the present invention the sterilant is ozone. In another aspect of the present invention an effective amount is defined as being sufficient to allow the composition to provide greater than a 1-log order reduction in the population of a target microorganism, within a predetermined amount of time.

Illustrative embodiments of the invention are described below. While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the spirit and scope of the invention as defined by the appended claims.

It will of course be appreciated that in the development of any such actual embodiment, numerous implementation-specific decisions must be made to achieve the developer's specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

As used in this invention, the term "packaging material" refers to a substance that is to be used to enclose, contain, protect, and transport a desired product. The packaging material in this invention is preferably used for food products, more preferably for liquid food products. Most preferably the packaging material in this invention is the multi-layer packaging material that is used in aseptic packaging equipment.

Aseptic packages are lightweight, multi-layer and energy efficient. They combine high-performance materials with high-performance construction and high-performance features. Typically, the package is about 70% paper (to provide stiffness, strength and the ability to hold a desired shape), about 25% low-density polyethylene (to seal the carton liquid-tight), and about 5% aluminum (to keep out light and oxygen). Together, these materials produce a container that safeguards the aseptically processed product inside. Typically, the package consists of about 6 individual layers, with the inner-most layer being polyethylene (to keep the exterior of the package dry), followed by a layer of paper, another of polyethylene, then aluminum, then one or two additional layers of polyethylene (to provide a printing surface for nutritional information and graphic design).

The multi-layer packaging material may be fed into the aseptic packaging machine as a sheet. This sheet is then treated with an antimicrobial agent to disinfect the surface. The sanitized sheet is then formed into a shape that is suitable to contain and protect the desired food product. The multi-layer packaging material may be fed into the aseptic packaging machine as a preformed tube. This tube is then treated with an antimicrobial agent to disinfect (primarily) the inner surface that is to come into contact with the food product. The tube is then filled with the food product and formed into a container. Those of skill in the art of aseptic packaging will recognize that this invention may be used on any form of aseptic packaging, and is not limited to these examples.

As used in this invention, the term "sterilant" refers to a physical or chemical agent or process capable of destroying all forms of life (including bacteria, viruses, fungi and spores) on inanimate surfaces.

As used in this invention, the term "effective amount" may refer to either an amount of sterilant provided to the packaging surface or a time during which the sterilant has been provided to the packaging surface to achieve sterilizing conditions. As will be recognized, an effective amount of sterilant depends on the relationship between the amount of sterilant used and the time period during which it is utilized. When the packaging surface is subjected to large amounts of sterilant, shorter exposure time periods may be appropriate to sterilize these objects.

One means of measuring the effectiveness of a sterilant in the industry, is to measure the reduction in the population of a target microorganism in a predetermined amount of time. The value known as the D value, or decimal reduction time value, is established as being the conditions required to kill 90% of the population of target microorganisms. This is known as a 1-log order reduction in the population of these target microorganisms. Therefore, if it takes two minutes at 60 °C to obtain a reduction of 90% in the target population, D60 = 2. Likewise a reduction of 99% in the target population is known as a 2-log order reduction, a reduction of 99.9% in the target population is known as a 3-log order reduction, and so on.

As used in this invention, the term "antimicrobial" refers to a physical or chemical agent capable of causing greater than 90% reduction (1-log order reduction) in the population of bacteria or spores of Bacillus species within 10 seconds at 60 °C. Typically Bacillus cereus or bacillus subtilis are used as target microorganisms in this procedure. The antimicrobial composition of the invention preferably provides greater than a 99% reduction (2-log order reduction), and more preferably greater than a 99.99% (4-log order reduction), and most preferably a 99.999% (5-log order reduction) in such a population preferably within 60 seconds at 60 °C, and more preferably within 10 seconds at 60 °C.

Existing hydrogen peroxide bath machines may be retrofitted to incorporate this invention. The ozone and liquid carbon dioxide will be sprayed using a nozzle to treat the packaging surface, instead of utilizing the bath arrangement that currently exists to contact hydrogen peroxide with the packaging surface. The ozone gas that is released, in combination with lower temperatures provided by liquid carbon dioxide, is expected to sanitize the packaging surface. This released gas will be contained within the portion of machine that is presently configured to enclose and capture the harmful effects due to hydrogen peroxide vapors that are not broken down into oxygen and water vapor. The air used for heating will be also be inherently sanitized due to presence of ozone in the circulating air, which is an added advantage to the process.

Existing hydrogen peroxide spray machines may be retrofitted to incorporate this invention. The ozone and liquid carbon dioxide will be sprayed using a nozzle to treat the packaging surface. The spray nozzles currently being used for hydrogen peroxide will be modified to spray ozone and liquid carbon dioxide. This ozone gas that is released, in combination with lower temperatures provided by liquid carbon dioxide, is expected to sanitize the packaging surface. This released gas will be contained within the portion of machine that is presently configured to enclose harmful effects due to hydrogen peroxide vapors that are not broken down into oxygen and water vapor. The air used for heating will also be inherently sanitized due to presence of ozone in the circulating air, which is an added advantage to the process.

Sterilants such as a combination of hydrogen peroxide and water, chlorine and water or ozonated water may not be used in the present invention. The presence of any water at the low temperatures of the cryogenic liquid that is used to transport the sterilant will result in the formation of ice at various points within application means. Due to the resulting temperature depression due to the rapid pressure drop through the spray head itself, this is likely the first place that such ice formation would take place. This would result in sprayer clogging, the avoidance of which is one object of the present invention.

## Claims

1. An antimicrobial composition comprising a liquid, other than water, and an effective amount of a sterilant.

2. The composition according to claim 1, wherein the liquid and the sterilant are devoid of any water.

3. The composition according to claim 1, wherein the liquid is liquid carbon dioxide.

4. The composition according to claim 1, wherein the liquid is a cryogenic fluid.

5. The composition according to claim 4, wherein said cryogenic fluid is selected from the group consisting of liquid oxygen, liquid nitrogen, liquid argon, liquid air, or any combination.

6. The composition according to claim 1, wherein said sterilant is ozone.

7. The composition according to claim 6, wherein said sterilant is in the gas phase.

8. The composition according to claim 6, wherein said sterilant is in the liquid phase.

9. The composition according to claim 1, wherein an effective amount is defined as being sufficient to allow the composition to provide greater than a 1-log order reduction in the population of a target microorganism, within a predetermined period of time.

10. The composition according to claim 1, wherein an effective amount is defined as being sufficient to allow the composition to provide greater than a 5-log order reduction in the population of a target microorganism, within a predetermined period of time.

11. The composition according to claim 9, wherein the target microorganism is selected from the group consisting of:
• *Staphylococcus aureus ,*
• *Clostridium perfringens,*
• *Salmonella,*
• *Clostridium botulinum,*
• *Vibrio parahaemolyticus,*
• *Bacillus cereus,*
• *Bacillus Subtilis,*
• *Campylobacter jejuni,*
• *Escherichia coli*, and
• *Listeria.*

12. The composition according to claim 10, wherein the target microorganism is selected from the group consisting of:
• *Staphylococcus aureus ,*
• *Clostridium perfringens*,
• *Salmonella,*
• *Clostridium botulinum,*
• *Vibrio parahaemolyticus*,
• *Bacillus cereus,*
• *Bacillus subtilis*,
• *Campylobacter jejuni,*
• *Escherichia coli*, and
• *Listeria.*

13. The composition according to claim 9, wherein the predetermined period of time is less than 60 seconds.

14. The composition according to claim 9, wherein the predetermined period of time is less than 10 seconds.

15. The composition according to claim 10, wherein the predetermined period of time is less than 60 seconds.

16. The composition according to claim 10, wherein the predetermined period of time is less than 10 seconds.

17. A method for the treatment of the surface of packaging material , comprising:
a. providing a packaging material,
b. providing an antimicrobial composition according to claim 1,
c. applying said composition to the surface of the packaging material with an application means, and
d. heating the surface of the packaging material with a heating means.

18. The method according to claim 17, wherein said application means is a spraying means.

19. The method according to claim 17, wherein the application means is to feed packaging material continuously into a bath comprising said antimicrobial composition.

20. The method according to claim 17, wherein said heating means is heated air.

21. The method according to claim 17, further comprising a gas recovery and exhaust means.
